# EUROPÄISCHE PATENTANMELDUNG

(11) **EP 0 822 221 A2**
(43) Veröffentlichungstag der Anmeldung: **04.02.1998**
(21) Anmeldenummer: 97112675.0
(22) Anmeldetag: 24.07.1997
(51) Int. Cl.: C08K 5/00

(54) **Neue Lichtstabilisatoren auf Basis von sterisch gehinderten Aminen**

(30) Priorität: 02.08.1996 DE 19631244
(71) Anmelder: Clariant GmbH, 65929 Frankfurt am Main (DE)
(72) Erfinder: Gaa, Karl, Dr., 89349 Burtenbach (DE); Zäh, Matthias, Dr., 86368 Gersthofen (DE); Mehrer, Mathias, Dr., 86456 Gablingen (DE); Pfahler, Gerhard, Dr., 86169 Augsburg (DE); Stährfeldt, Thomas, Dr., 86356 Neusäss (DE)

(57) **Zusammenfassung**

Die vorliegende Erfindung bezieht sich auf neue UV-Stabilisatoren der allgemeinen Formel (I), 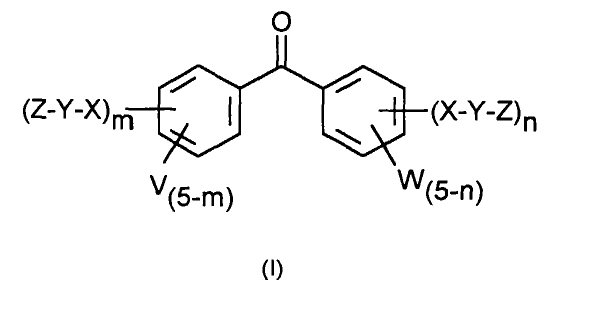 wobei
die Substituenten die in der Beschreibung offenbarte Bedeutung besitzen.

Diese Verbindungen eignen sich in hervorragender Weise zum Stabilisieren von organischem Material wie Kunststoffe, Lacke, Anstrichmittel und Öle gegen die Einwirkung von Licht, Strahlung, Sauerstoff und Wärme.

## Beschreibung

Es ist bekannt, daß organische Materialien durch energiereiche Strahlung, Wärme oder Sauerstoff geschädigt werden. Die Oberflächenschicht organischer Materialien ist diesen schädigenden Einflüssen meist in besonderem Maße ausgesetzt. Das Problem macht sich besonders bei dünnen Artikeln, wie Fasern, Bändchen und Folien bemerkbar, führt aber auch bei dickwandigeren Artikeln zu einer Schädigung (z. B. Versprödung) besonders der Oberflächenschicht.

Es gibt bereits viele Druckschriften, die Verbindungen zur Stabilisierung von organischem Material beschreiben. Ein Teil davon betrifft Verbindungen auf der Basis von 2,2,6,6-Tetraalkylpiperidin. Diese Stabilisatoren müssen besonders in der Oberflächenschicht des organischen Materials in ausreichender Konzentration vorliegen, um einen effektiven Schutz zu bewirken. Die niedermolekularen Vertreter aus der Substanzklasse der 2,2,6,6-Tetraalkylpiperidine haben den Vorteil, schnell in die Oberflächenschicht zu migrieren und dort ihren schützenden Einfluß zu entfalten. Sie haben allerdings den gravierenden Nachteil, daß sie eine zu hohe Flüchtigkeit besitzen und aus dem organischen Material sehr gut extrahierbar sind. Die höhermolekularen Vertreter dieser Substanzklasse sind zwar nicht so gut extrahierbar, migrieren jedoch deutlich langsamer. In der Technik wird diesem Problem dadurch begegnet, daß man eine Mischung von niedermolekularen (schnell migrierenden) und hochmolekularen (langsam migrierenden) Stabilisatoren zum Einsatz bringt.

Aus DE-A-4 327 297 sind Verbindungen bekannt, die sich von 2,2,6,6-Tetraalkylpiperidinen herleiten und nach Einwirkung von ultraviolettem Licht in eine extraktionsstabile Form umgewandelt werden. Bei der die Migrationsgeschwindigkeit erniedrigenden Komponente handelt es sich in DE-A-4 327 297 um ein Derivat der Zimtsäure. Sie wird dafür verantwortlich gemacht, daß der Stabilisator eine kovalente Bindung zum organischen Material eingeht.
In EP-A-389 427 werden Verbindungen beschrieben, welche in demselben Molekül zugleich ein Derivat des 2,2,6,6-Tetraalkylpiperidins und ein Benzophenon enthalten. Dort werden jedoch lediglich solche Benzophenone offenbart, die α-Hydroxylgruppen besitzen und so als UV-Licht-Absorber wirken.

Überraschenderweise konnte gefunden werden, daß geeignet substituierte Vertreter des Benzophenons (a) in organischen Materialien "schnell" migrieren und so in die Oberflächenschicht des Materials schnell einwandern, (b) nach Einwirkung von Licht der Wellenlänge von 280 - 700 nm (bevorzugt unter Einwirkung von Licht der Wellenlänge von 300 - 400 nm) in eine weniger gut bzw. gar nicht mehr extrahierbare Form gebracht werden und (c) auch in dieser neuen, nicht mehr extrahierbaren Form organische Materialien weiterhin vor den oben erwähnten schädigenden Einflüssen des ultravioletten Lichts wirkungsvoll schützen.

Gegenstand der Erfindung sind somit UV-Stabilisatoren der allgemeinen Formel (I), 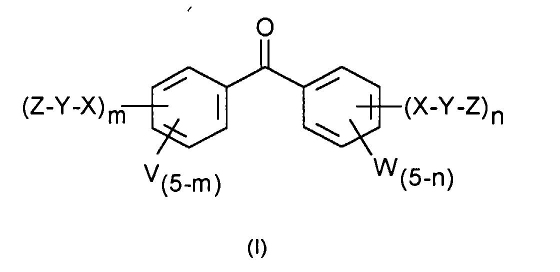 worin
die im aromatischen Ring zur Carbonylgruppe α-ständigen Substituenten nicht OH bedeuten dürfen,
- m und n: unabhängig voneinander 0, 1 oder 2 bedeuten,
- V oder W: unabhängig voneinander H, Hal, NO₂, OH, OR¹, CN, SR¹, C₁-C₁₈-, vorzugsweise C₁-C₁₀-, insbesondere C₁-C₄-Alkyl, C₃-C₁₀-, vorzugsweise C₅-C₆-Cycloalkyl, unsubstituiertes oder mit OH, OR¹, Hal oder NR¹R² substituiertes C₆-C₁₄-Aryl oder C₇-C₁₀-Arylalkyl, einen heteroaromatischen Rest mit 5-15, vorzugsweise 6-10 C-Atomen, NR¹R² oder COOR¹ bedeuten,
- R¹ und R²: H, C₁-C₁₈-, vorzugsweise C₁-C₁₀-, insbesondere C₁-C₄-Alkyl, C₃-C₁₀-, vorzugsweise C₅-C₆-Cycloalkyl, unsubstituiertes oder mit OH, OR¹ oder Hal substituiertes C₆-C₁₄-Aryl oder C₇-C₁₀-Arylalkyl, oder einen heteroaromatischen Rest mit 5-15, vorzugsweise 6-10 C-Atomen bedeuten,
- X: einen zweibindigen Rest -O-, -S-, oder -C(O)- bedeutet,
- Y: für X = O oder S -CH₂-C(O)-, -C(O)-, -C(O)-C(O)-, -CH₂-CHR¹-, -(CH₂- CH₂-O)ₙ- (mit n = 1 - 8), vorzugsweise -C(O)- oder -CH₂-C(O)-, oder einen Rest 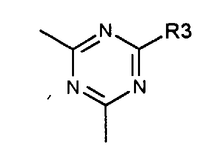 bedeutet, wobei R³ NR¹R², OR¹, Halogen oder Z bedeutet,
- Y: für X = -C(O)- eine direkte Bindung zu Z bedeutet,
- Z: einen der folgenden Reste bedeutet, 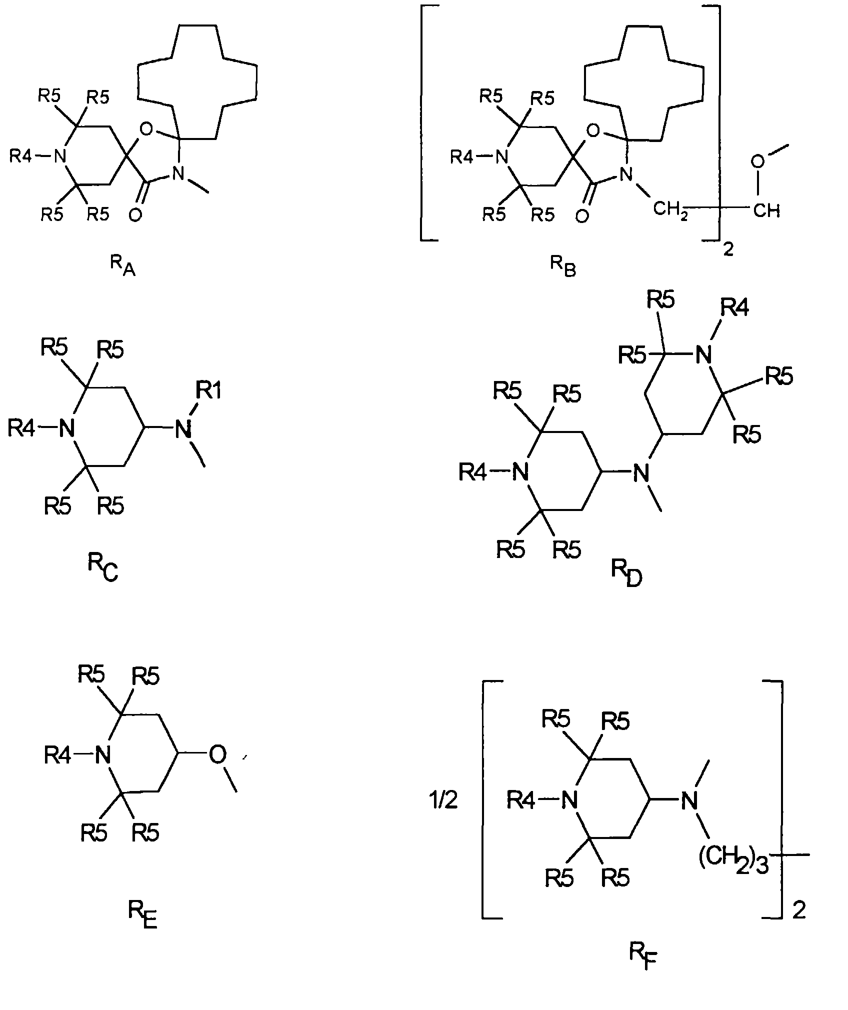
vorzugsweise R_{C} oder R_{E},
worin
- R⁴: Wasserstoff, C₁-C₂₀-, vorzugsweise C₁-C₁₀-, insbesondere C₁-C₅-Alkyl, ein Oxyl-Radikal, OH, NO, CH₂CN, unsubstituiertes oder mit OH, OR¹, Hal oder NR¹R² substituiertes C₆-C₁₄-Aryl oder C₇-C₁₀-Arylalkyl, Allyl, C₁-C₃₀-, vorzugsweise C₁-C₁₀-, insbesondere C₁-C₅-Alkyloxy, C₅-C₁₂-, vorzugsweise C₆-C₉-Cycloalkyloxy, C₃-C₁₀-, vorzugsweise C₄-C₈-Alkenyl, C₃-C₆-Alkinyl, C₁-C₁₀-, vorzugsweise C₁-C₅-Acyl, Halogen,
- R⁵: Wasserstoff oder C₁-C₄-Alkyl, vorzugsweise Methyl, bedeutet.

Sehr gut geeignet sind auch Stabilisatoren, worin
die im aromatischen Ring zur Carbonylgruppe α-ständigen Substituenten nicht OH bedeuten dürfen,
- m und n: unabhängig voneinander 0, 1 oder 2 bedeuten,
- V oder W: unabhängig voneinander H, NO₂, OH, C₁-C₁₀-Alkyl, C₅-C₆-Cycloalkyl, unsubstituiertes oder mit OH oder NR¹R² substituiertes C₆-C₁₄-Aryl oder C₇-C₁₀-Arylalkyl, einen heteroaromatischen Rest mit 6-10 C-Atomen, NR¹R² oder COOR¹ bedeuten,
- R¹ und R²: H, C₁-C₁₀-Alkyl, C₅-C₆-Cycloalkyl, unsubstituiertes oder mit OH substituiertes C₆-C₁₄-Aryl oder C₇-C₁₀-Arylalkyl, oder einen heteroaromatischen Rest mit 6-10 C-Atomen bedeuten,
- X: einen zweibindigen Rest -O- oder -C(O)- bedeutet,
- Y: für X = O -CH₂-C(O)-, -C(O)- oder -CH₂-CHR¹- bedeutet,
- Z: den Rest R_{A}, R_{C}, R_{D} oder R_{E} bedeutet, worin
- R⁴: Wasserstoff, C₁-C₁₀-Alkyl, ein Oxyl-Radikal, OH, NO, unsubstituiertes oder mit OH oder NR¹R² substituiertes C₆-C₁₄-Aryl oder C₇-C₁₀-Arylalkyl, Allyl, C₁-C₁₀-Alkyloxy, C₆-C₉-Cycloalkyloxy, C₄-C₈-Alkenyl, C₃-C₆-Alkinyl, C₁-C₅-Acyl, Halogen, und
- R⁵: Wasserstoff oder C₁-C₄-Alkyl bedeutet.

Besonders geeignete UV-Stabilisatoren sind Verbindungen der Formel I, worin die im aromatischen Ring zur Carbonylgruppe α-ständigen Substituenten nicht OH bedeuten dürfen,
- m und n: unabhängig voneinander 0, 1 oder 2 bedeuten,
- V oder W: unabhängig voneinander H, NO₂, C₁-C₄-Alkyl, NR¹R² oder COOR¹ bedeuten,
- R¹ und R²: H, C₁-C₄-Alkyl, unsubstituiertes oder mit OH substituiertes C₆-C₁₄-Aryl oder C₇-C₁₀-Arylalkyl bedeuten,
- X: einen zweibindigen Rest -O- oder -C(O)- bedeutet,
- Y: für X = -O- -C(O)- oder CH₂-C(O)- bedeutet,
- Y: für X = -C(O)- eine direkte Bindung zu Z bedeutet,
- Z: einen Rest der Formel R_{C} oder R_{E} bedeutet, worin
- R⁴: Wasserstoff, OH, NO, C₁-C₅-Alkyl, C₁-C₅-Alkyloxy,C₁-C₅-Acyl und
- R⁵: Methyl bedeutet.

Die Erfindung bezieht sich auch auf die Herstellung dieser Verbindungen (I). Die erfindungsgemäßen Verbindungen können grundsätzlich auf zwei verschiedenen Wegen aufgebaut werden.

Zum einen kann man die erfindungsgemäße Verbindung in einer Weise aufbauen, daß ein Benzophenon (II) mit einem oder mehreren Äquivalenten eines geeigneten Reagenzes R in ein reaktives Molekül (III) überführt wird:

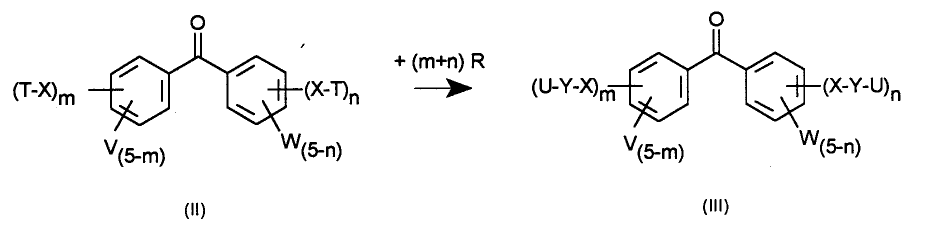

Bedeutung von T:
Für den Fall, daß X = -O-, -S- bedeutet, so gilt: T = Wasserstoff.
Für den Fall, daß X = -C(O)- bedeutet, so gilt: T = OR¹, NR¹R², -O-C(O)-R¹, OH.

Bedeutung von R:
Für den Fall, daß X-T = OH oder SH bedeutet:
   - R bedeutet: Hal-CH₂-C(O)-U,
   U-C(O)-U,
   U-C(O)-C-(O)-U,
   Ethylenoxid
   Hal-CH₂-CHR¹-Hal, 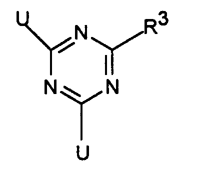
wobei R³ = NR¹R², OR¹, Halogen oder Z bedeutet.
Bedeutung von U: U = Halogen, OR¹, NR¹R².
Die übrigen Substituenten sind wie in Formel I definiert.

### Beispiele für die Verbindung (III) sind:

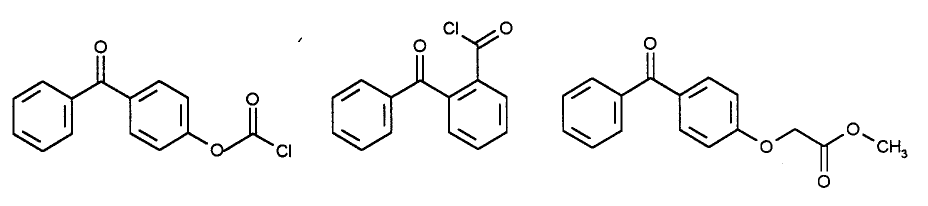

Bei dem Reagenz R handelt es sich z. B. um 2,4,6-Trichlor-s-triazin (= Cyanurchlorid), Phosgen, Bromessigsäurealkylester, Chloressigsäurealkylester, Chlorameisensäuremethylester, Oxalsäure, Oxalsäuredihalogenid, Oxalsäuredialkylester, Oxalsäurehalogenidmonoester, Ethylenoxid, Thionylchlorid (SOCl₂), Phosphor(III)halogenid (PCl₃ oder PBr₃), Halogenwasserstoff (HCl, HBr), elementares Chlor.

In einem zweiten Schritt wird die Verbindung (III) mit Z-H zu der erfindungsgemäßen Verbindung (I) umgesetzt. Zur Reaktion kann auch das Anion Z⁻ verwendet werden, welches vorzugsweise aus der Verbindung Z-H hergestellt wird (mit Z = in Formel I beschriebene Bedeutung und H = Wasserstoff). Falls es sich in Verbindung (II) bei -X-T um -C(O)OR¹, -C(O)NR¹R², -C(O)-O-C(O)-R¹ handelt, so kann auch bereits die Verbindung (II) mit Z-H oder mit dem Anion Z⁻ zu der Zielverbindung (I) umgesetzt werden.

Alternativ kann der Weg beschritten werden, zuerst ein literaturbekanntes Derivat des 2,2,6,6-Tetraalkylpiperidins, wie z. B. eine der Verbindungen Z-H oder Z-M mit (M = z. B. Na, K ...), mit einem Reagenz (U-Y-U) zu dem Intermediat Z-Y-U umzusetzen. Z und H haben die weiter oben angegebene Bedeutung.
Verbindung Z-Y-U wird in dieser Herstellungsvariante in einer anschließenden Reaktion mit einem geeignet substituierten Derivat des Benzophenons (= Verbindungen der Formel II mit -XT = -OH oder -SH) zu den erfindungsgemäßen Verbindungen (I) umgesetzt.

Die Umsetzungen erfolgen in einem protischen oder aprotischen, organischen Lösemittel, vorzugsweise einem Kohlenwasserstoff, insbesondere aromatischen Kohlenwasserstoffen wie beispielsweise Toluol, Xylol oder in Gemischen hieraus oder in Tetrahydrofuran. Eine weitere Möglichkeit besteht darin, eine der Reaktionskomponenten im Überschuß als Lösemittel zu verwenden.

Die erfindungsgemäßen Verbindungen eignen sich in hervorragender Weise zum Stabilisieren von organischem Material gegen die Einwirkung von Licht, Strahlung, Sauerstoff und Wärme. Sie werden dem zu stabilisierenden organischen Material in einer Konzentration von 0,001 bis 5 Gew.-%, vorzugsweise von 0,02 bis 1,0 Gew.-%, bezogen auf das organische Material, vor, während oder nach seiner Herstellung zugesetzt.

Unter organischem Material sind beispielsweise Vorprodukte für Kunststoffe, Lacke, Anstrichmittel und Öle, insbesondere jedoch Kunststoffe, Lacke, Anstrichmittel und Öle selbst, zu verstehen.

Gegenstand der vorliegenden Erfindung sind außerdem gegen die Einwirkung von Licht, Strahlung, Sauerstoff und Wärme stabilisierte organische Materialien, insbesondere Kunststoffe, Lacke, Anstrichmittel und Öle, welche die erfindungsgemäßen Verbindungen in den oben angegebenen Konzentrationen enthalten. Zu diesen organischen Materialien gehören beispielsweise Stoffe, wie sie beschrieben sind auf den Seiten 13-18 der Europäischen Patentanmeldung 95 109 778.1-1270, auf die hier ausdrücklich Bezug genommen wird.

Das durch die erfindungsgemäßen Verbindungen stabilisierte organische Material kann gegebenenfalls noch weitere Additive enthalten, beispielsweise Antioxidantien Lichtstabilisierungsmittel, Metalldesaktivatoren, antistatische Mittel, flammhemmende Mittel, Pigmente und Füllstoffe. Antioxidantien und Lichtstabilisatoren, die neben den erfindungsgemäßen Verbindungen zugesetzt werden, sind beispielsweise Verbindungen auf der Basis sterisch gehinderter Amine oder sterisch gehinderter Phenole oder Schwefel oder Phosphor enthaltende Costabilisatoren. Als geeignete zusätzliche Additive kommen beispielsweise Verbindungen in Betracht, wie sie beschrieben sind auf den Seiten 18-29 der Europäischen Patentanmeldung 95 109 778.1-1270, auf die hier ausdrücklich Bezug genommen wird.
Als weitere geeignete Additive kommen zusätzlich in Betracht: 2,2',2''-Nitrilo[triethyl-tris(3,3',5,5'-tetra-tert.butyl-1,1'-biphenyl-2,2'-diyl)phosphit], Bis[2-methyl-4,6-bis(1,1-dimethylethyl)phenol]phosphorigsäure-ethylester, sekundäre Hydroxylamine wie z. B. Distearylhydroxylamin oder Dilaurylstearylamin, Zeolithe wie z. B. DHT 4A, Oxide und Hydroxide von Aluminium, Zink, Alkali- und Erdalkalimetalle, Al-, Ca-, Mg-, Zn-Stearat, wobei für einzelne Anwendungen besonders feinkörniges Material speziell geeignet ist, Kondensationsprodukt aus N,N'-Bis[(4,6-di(4-n-butylamino-2,2,6,6-tetramethylpiperid-4-yl)1,3,5-triazin-2-yl]-3- aminopropyl-ethylen-1,2-diamin und 2,4-Dichlor-6-(4-n-butylamino-2,2,6,6-tetramethylpiperid-4-yl)1,3,5-triazin, 1,3,-2,4-Di(benziliden)-D-sorbitol, 1,3-2,4-Di-(4-tolyliden)-D-sorbitol, 1,3-2,4-Di(4-ethylbenziliden)-D-sorbitol.

Mischungsbestandteil können auch Hydrotalcite sein, die beschrieben werden können nach der allgemeinen Formel

[(M²⁺)₁₋ₓ (M³⁺)ₓ (OH)₂ (Aⁿ⁻)_{x/n} yH₂O],

wobei
- (M²⁺): Mg, Ca, Sr, Ba, Zn, Pb, Sn, Ni,
- (M³⁺): Al, B, Bi,
- Aⁿ: ein Anion der Wertigkeit n,
- n: eine ganze Zahl von 1 - 4,
- x: einen Wert zwischen 0 und 0,5,
- y: einen Wert zwischen 0 und 2, und
- A: OH⁻, Cl⁻, Br⁻, I⁻, ClO₄⁻, CH₃COO⁻, C₆H₅COO⁻, CO₃²⁻, SO₄²⁻, (OOC-COO)²⁻, (CHOHCOO)₂²⁻, (CHOH)₄CH₂OHCOO⁻, C₂H₄(COO)₂²⁻, (CH₂COO)₂²⁻, CH₃CHOHCOO⁻, SiO₃²⁻, SiO₄⁴-, Fe(CN)₆³⁻, Fe(CN)₆⁴⁻, BO₃³⁻, PO₃³⁻, HPO₄²⁻, bedeutet.

Bevorzugt eingesetzt werden Hydrotalcite, in welchen (M²⁺) für (Ca²⁺), (Mg²⁺) oder eine Mischung aus (Mg²⁺) und (Zn²⁺); (Aⁿ⁻) für CO₃²⁻, BO₃³⁻, PO₃³⁻ stehen. Weiterhin können auch Hydrotalcite, die mit der Formel

[(M²⁺)ₓ (Al³⁺)₂ (OH)_{2x+6nz} (Aⁿ⁻)₂ yH₂O]

beschrieben werden können, eingesetzt werden.
Hier steht (M²⁺) für Mg²⁺ oder Zn²⁺, bevorzugterweise jedoch Mg²⁺.
(Aⁿ⁻) steht für ein Anion, besonders aus der Gruppe CO₃²⁻, (OOC-COO)²⁻, OH⁻ und S²⁻, wobei n die Wertigkeit des lons beschreibt.
y steht für eine positive Zahl, bevorzugterweise zwischen 0 und 5, insbesonders zwischen 0,5 und 5;
x und z haben positive Werte, die bei x bevorzugt zwischen 2 und 6 liegen und bei z kleiner als 2 sein sollten. Besonders bevorzugt sind die Hydrotalcite der folgenden Formeln anzusehen:

   Al₂O₃ · 6MgO · CO₂ · 12H₂O , Mg_{4.5}Al₂(OH)₁₃ · CO₃ · 3.5H₂O , 4MgO · Al₂O₃ · CO₂ · 9H₂O , 4MgO · Al₂O₃ · CO₂ · 6H₂O , ZnO · 3MgO · Al₂O₃ · CO₂ · 8-9H₂O , ZnO · 3MgO · Al₂O₃ · CO₂ · 5-6H₂O , Mg_{4.5}Al₂(OH)₁₃ · CO₃ .

Hydrotalcite werden im Polymer bevorzugt in einer Konzentration von 0,01 bis 5 Gew.%, besonders von 0,2 bis 3 Gew.%, bezogen auf die gesamte Polymerzubereitung, eingesetzt.

Die Additive werden nach allgemein üblichen Methoden in die organischen Polymere eingearbeitet. Die Einarbeitung kann beispielsweise durch Einmischen oder Aufbringen der Verbindungen und gegebenenfalls weiterer Additive in oder auf das Polymere unmittelbar nach der Polymerisation oder in die Schmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglicher Verdunstung des Lösemittels, kann die Einarbeitung erfolgen. Die Verbindungen sind auch wirksam, wenn sie in ein bereits granuliertes Polymer nachträglich in einem gesonderten Verarbeitungsschritt eingebracht werden. Die erfindungsgemäßen Verbindungen der Formel (I) können auch in Form eines Masterbatches, der diese Verbindungen beispielsweise in einer Konzentration von 1 bis 75, vorzugsweise 2,5 bis 30 Gew.-% enthält, den zu stabilisierenden Polymeren zugesetzt werden.

### Beispiele

Die nachfolgenden Beispiele sollen die Erfindung näher erläutern. Alle Verbindungen konnten anhand ihrer ¹H- bzw. ¹³C-NMR-Spektren eindeutig identifiziert werden. Die Herkunft der Ausgangsmaterialien ist in den entsprechenden Tabellen ausgewiesen.

### Beispiele 1.6:

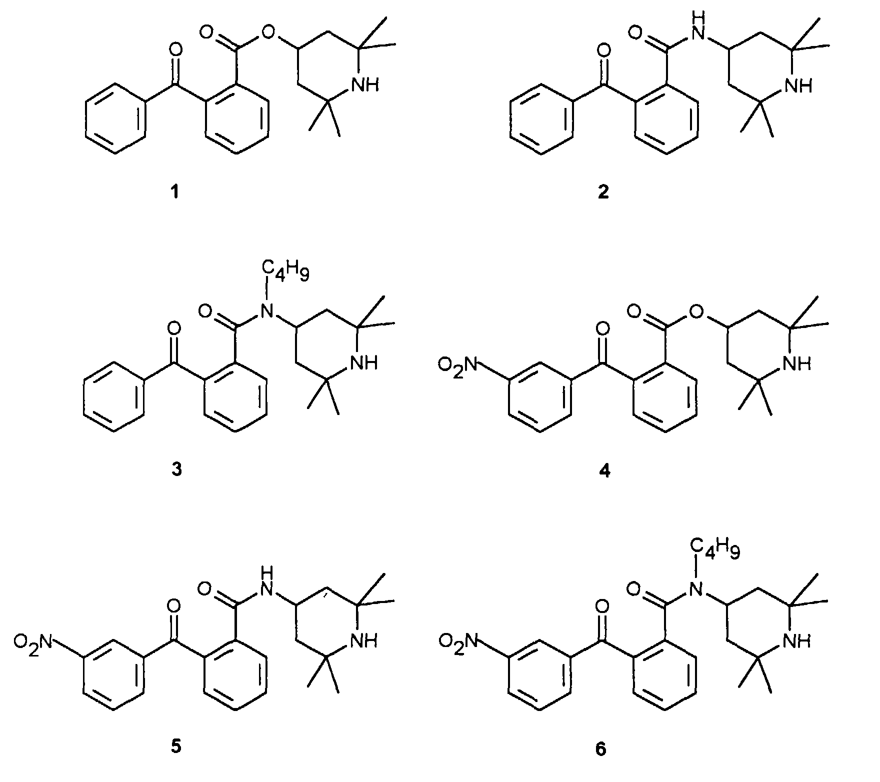

### Allgemeine Vorschrift für die Herstellung der Verbindungen 1 - 6:

Die Umsetzung wird unter Stickstoff und unter Ausschluß von Luft und Wasser durchgeführt.
Eine Menge von 25-100 mmol der Verbindung A, B oder C (Tabelle 1) und ein 10%iger Überschuß von Triethylamin werden in 150 ml Toluol vorgelegt. Eine, bezogen auf A, B oder C, äquimolare Menge eines Säurechlorides D oder E wird in 30 ml Toluol gelöst und in die vorgelegte Lösung bei 22 °C langsam zugegeben. Nach 3 h Rühren bei 109 °C fällt ein heller Niederschlag aus. Die Reaktionsmischung wird auf Wasser gegossen, der Niederschlag löst sich auf. Die organische Phase wird mit Wasser gewaschen, mit Natriumsulfat getrocknet und von diesem abfiltriert. Die flüchtigen Bestandteile werden im Vakuum entfernt, der Rückstand wird bei 80 °C im Vakuum getrocknet.

**Tabelle 1**

| Verbindg.-Nr. | Edukt* | Menge NEt₃** | Säurechlorid*** | Ausbeute | Charakterisierung | λ ₘₐₓ**** |
|---|---|---|---|---|---|---|
| **1** (M = 365,51) | **A**, 15,7 g (100 mmol) | 11,1 g (110 mmol) | **D**, 24,5 g (100 mmol) | 22,8 g (63%) | hellgelbes Öl | 278 nm (vs) 325 nm (s) |
| **2** (M = 364,53) | **B**, 7,8 g (50 mmol) | 5,6 g (55 mmol) | **D**, 12,2 g (50 mmol) | 14,4 g (79%) | gelbe Krist. Fp.=200-01°C | 255 nm (vs) 288 nm (s) |
| **3** (M = 420,65) | **C**, 21,2 g (100 mmol) | 11,1 g (110 mmol) | **D**, 24,5 g (100 mmol) | 39,6 g (94%) | hellgelbes Öl | 254 nm (vs) 340 nm (s) |
| **4** (M = 410,51) | **A**, 7,2 g (46 mmol) | 5,1 g (50 mmol) | **E**, 13,3 g (46 mmol) | 15,3 g (81%) | weiß, wachsartig | 290 nm (vs) 350 nm (s) |
| **5** (M = 409,53) | **B**, 7,2 g (46 mmol) | 5,1 g (50 mmol) | **E**, 13,3 g (46 mmol) | 14,4 g (76%) | gelbe Krist. Fp.=216-18°C | 257 nm (vs) 310 nm (s,sh) |
| **6** (M = 465,65) | **C**, 5,1 g (24 mmol) | 2,7 g (27 mmol) | **E**, 7,0 g (24 mmol) | 7,0 g (63%) | hellgelbes Öl | 235 nm (vs) 270 nm (s) 340 nm(m,sh) |
| A = 2,2,6,6-Tetramethylpiperidin-4-ol* (M = 157,29) B = 2,2,6,6-Tetramethyl-4-piperidinamin* (M = 156,31) C = N-Butyl-2,2,6,6-tetramethyl-4-piperidinamin* (M = 212,43) D = Benzophenon-2-carbonsäurechlorid***, (M = 244,68) E = 3-Nitrobenzophenon-2 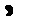 -carbonsäurechlorid***, M = Molgewicht; vs = very strong; s = strong; sh = shoulder; m = medium; w = weak | | | | | | |

| | | | | | | |
|---|---|---|---|---|---|---|
| * Bezogen von der Fa. HÜLS AG, Marl. | | | | | | |
| ** Bezogen von der Fa. Aldrich GmbH & Co KG, Steinheim. | | | | | | |
| *** Nach einer gängigen Methode selbst hergestelltes Produkt (org. Carbonsäure, umgesetzt mit SOCl₂); Bei dem Edukt von D handelt es sich um Benzophenon-2-carbonsäure** Bei dem Edukt von E handelt es sich um 3-Nitrobenzophenon-2 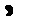 -carbonsäure, die nach einer gängigen Methode (Houben-Weyl X / 1, 471) hergestellt wurde (organische Carbonsäure, bezogen von der Fa. Aldrich GmbH & Co KG, Steinheim, umgesetzt mit Nitriersäure). | | | | | | |
| **** gemessen in Ethanol | | | | | | |

### Zwischenprodukte 7 und 8:

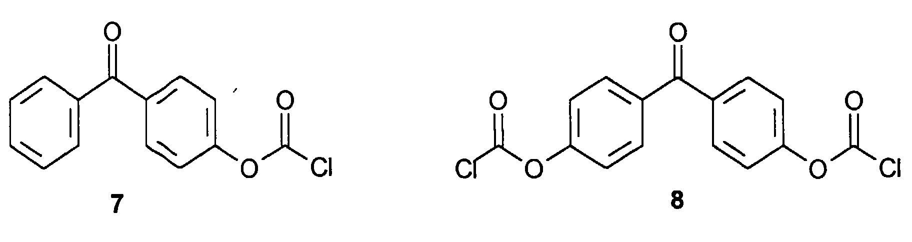

### Allgemeine Vorschrift: für die Herstellung der Verbindungen 7 und 8:

Etwa 50 - 120 mmol der Verbindung F oder G (vgl. Tabelle 2) und ein leichter Überschuß an N,N-Dimethylanilin werden in einer Mischung aus 150 ml Toluol und 50 ml Tetrahydrofuran vorgelegt. Über 16 Stunden wird bei 50 °C ein leichter Strom Phosgen durch die Lösung geleitet. Nach weiteren 2 Stunden Rühren bei 80 °C wird die Lösung auf Wasser gegossen. Die Mischung wird mit 10 %iger HCl auf pH 1 gebracht und mit 50 ml Methylenchlorid versetzt. Die organische Phase wird mit 10 %iger HCl gewaschen, abgetrennt, mit Natriumsulfat getrocknet, von diesem abfiltriert und im Vakuum vom Lösungsmittel befreit. Der Chlorameisensäureester wird in Form eines hellen, kristallinen Feststoffs isoliert.

### Beispiele 9 - 12:

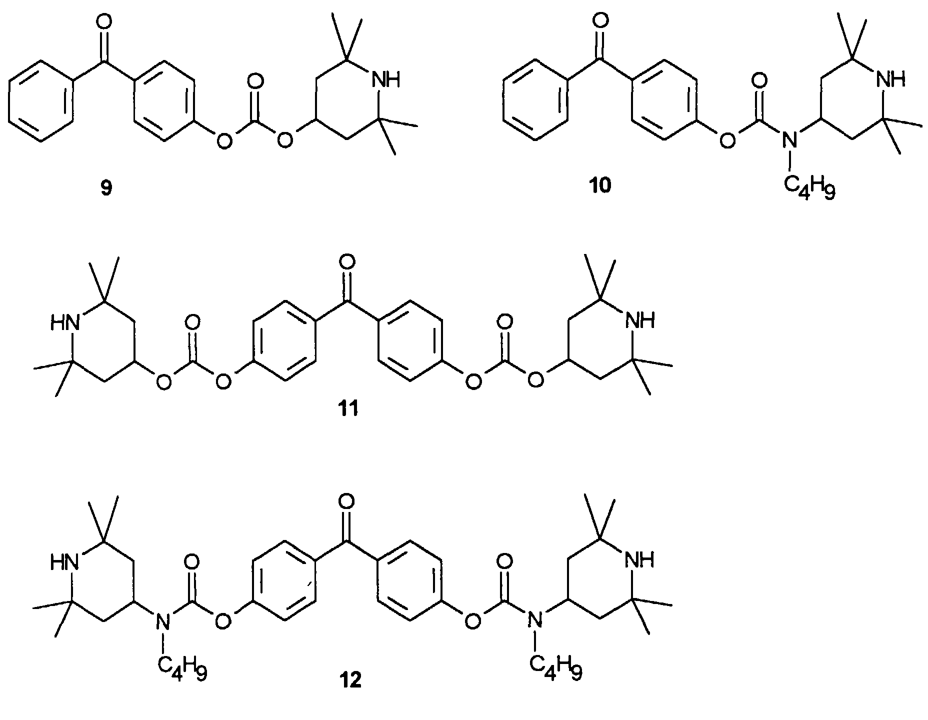

### Allgemeine Vorschrift: für die Herstellung der Verbindungen 9 - 12:

20-60 mmol der Verbindung A oder C (vgl. Tabelle 2) und ein leichter Überschuß eines Amins werden in 150 ml Toluol vorgelegt. Der Chlorameisensäureester (7 bzw. 8) wird in 30 ml Toluol gelöst und in die vorgelegte Lösung bei 20 °C unter Eiskühlung langsam zugegeben. Nach 2 h Rühren bei 22 °C und 1 h bei 107 °C wird die Reaktionsmischung auf Wasser gegossen und mit 50 ml Methylenchlorid versetzt. Die organische Phase wird mit Wasser gewaschen, mit Natriumsulfat getrocknet und filtriert. Die flüchtigen Bestandteile werden im Vakuum entfernt, der Rückstand wird bei 80 °C im Vakuum getrocknet.

**Tabelle 2**

| Verb.- Nr. | 1. Edukt | 2. Edukt | Amin | Ausbeute | Charakterisierung | λ ₘₐₓ*** |
|---|---|---|---|---|---|---|
| **7** (M = 260,68) | **F**, 9,5 g (48 mmol) | --- | **H**, 7,3 g (60 mmol) | 12,3 g (98%) | hellgrün, krist. Fp. = 64 -66°C | --- |
| **8** (M = 339,14) | **G**, 25,0 g (117 mmol) | --- | **H**, 30,5 g (252 mmol) | 33,4 g (84%) | hellgrau, krist. Fp.=106-108°C | --- |
| **9** (M = 381,51) | **A**, 6,3 g (40 mmol) | **7**, 10,5 g (40 mmol) | **I**, 3,8 g (48 mmol) | 8,2 g (53%) | weiß, krist. Fp.= 241-242°C | 255 nm (vs) |
| **10** (M = 436,65) | **C**, 4,9 g (23 mmol) | **7**, 6,0 g (23 mmol) | **J**, 2,6 g (26 mmol) | 7,3 g (74%) | gelbes Öl | 258 nm (vs) |
| **11** (M = 580,80) | **A**, 9,9 g (63 mmol) | **8**, 10,2 g (30 mmol) | **J**, 6,7 g (66 mmol) | 10,1 g (57%) | bernsteinfarbenes Öl | 265 nm (vs) |
| **12** (M = 691,07) | **C**, 13,4 g (63 mmol) | **8**, 8,4 g (25 mmol) | **J**, 6,7 g (66 mmol) | 10,5 g (60%) | bernsteinfarbenes Öl | 262 nm(vs) |
| A = 2,2,6,6-Tetramethylpiperidin-4-ol** (M = 157,29) B = N-Butyl-2,2,6,6-tetramethyl-4-piperidinamin** (M = 212,43) C = N-Butyl-2,2,6,6-tetramethyl-4-piperidinamin* (M = 212,43) F = 4-Hydroxybenzophenon* G = 4,4 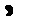 -Dihydroxybenzophenon* H = N,N-Dimethylanilin* I = Pyridin* J = Triethylamin* | | | | | | |

| | | | | | | |
|---|---|---|---|---|---|---|
| * Bezogen von der Fa. Aldrich GmbH & Co KG, Steinheim. | | | | | | |
| ** Bezogen von der Fa. HÜLS AG, Marl. | | | | | | |
| *** gemessen in Ethanol | | | | | | |

### Zwischenprodukte 13 - 19:

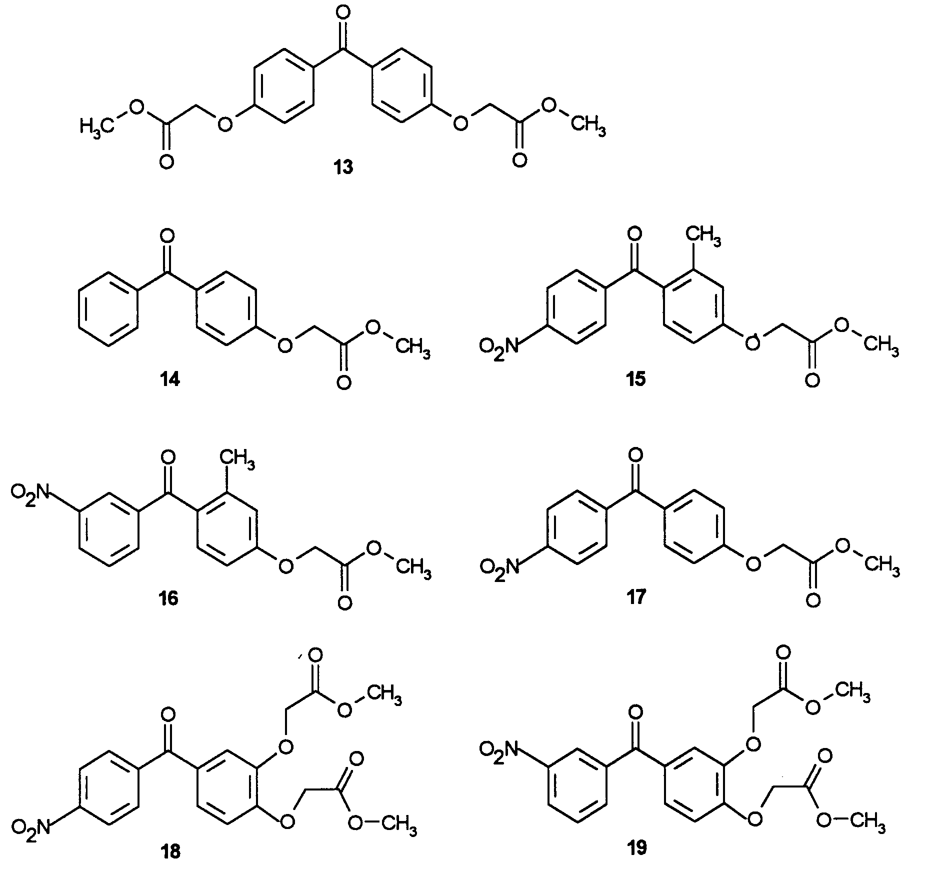

### Allgemeine Vorschrift für die Herstellung der Zwischenprodukte 13-19:

Die Umsetzung wird unter Stickstoff und unter Ausschluß von Luft und Wasser durchgeführt.
40-100 mmol der Verbindung K - Q (vgl. Tabelle 3) werden in 100 ml wasserfreiem Tetrahydrofuran vorgelegt. Ein 10%iger Überschuß an Natriumhydrid (80%ig in Paraffin) wird bei 20 °C portionsweise zugegeben, wobei die Lösung leicht aufschäumt und ein Niederschlag ausfällt. Nach 0,5 h Nachrühren ist der Vorgang beendet und eine äquimolare Menge an Bromessigsäuremethylester wird langsam zugegeben. Nach 8 h Nachrühren bei 66 °C hellt sich die Farbe der Suspension deutlich auf. Die flüchtigen Bestandteile werden im Vakuum entfernt, der Rückstand mit 50 ml Methylenchlorid versetzt und dreimal mit je 50 ml Wasser ausgeschüttet. Die organische Phase wird mit Natriumsulfat getrocknet, von diesem abfiltriert und im Vakuum werden die flüchtigen Bestandteile entfernt.

**Tabelle 3**

| Versuchs-Nr. | Edukt | NaH (80%ig in Paraffin)* | Bromessigsäuremethylester* | Ausbeute | Charakterisierung |
|---|---|---|---|---|---|
| **13** (M = 358,4) | **K**, 17,1 g (80 mmol) | 5,3 g (176 mmol) | 25,7 g (168 mmol) | 26,1 g (91%) | farblos,krist. Fp.= 178-179°C |
| **14** (M = 270,3) | **L**, 19,8 g (100 mmol) | 3,3 g (111 mmol) | 16,1 g (105 mmol) | 23,7 g (88%) | gelb, kristallin Fp.= 99-101°C |
| **15** (M = 327,3) | **M**, 15,4 g (60 mmol) | 2,0 g (66 mmol) | 9,2 g (60 mmol) | 13,5 g (68%) | beige, kristallin Fp.= 132-133°C |
| **16** (M = 329,3) | **N**, 25,7 g (100 mmol) | 3,3 g (110 mmol) | 15,3 g (100 mmol) | 25,3 g (77%) | braunes Öl, It. ¹H NMR rein |
| **17** (M = 315,1) | **O**, 24,3 g (100 mmol) | 3,3 g (110 mmol) | 15,3 g (100 mmol) | 20,0 g (63 %) | hellgelb, krist. Fp.= 136°C |
| **18** (M = 403,4) | **P**, 25,9 g (100 mmol) | 6,2 g (220 mmol) | 30,6 g (200 mmol) | 32,3 g (80%) | goldgelb, krist. Fp.= 149-150°C |
| **19** (M = 403,4) | **Q**, 11,7 g (45 mmol) | 3,0 g (100 mmol) | 13,8 g (90 mmol) | 15,9 g (88%) | goldgelbes Öl It. ¹H NMR rein |
| K = 4,4 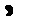 -Dihydroxybenzophenon* L = 4-Hydroxybenzophenon* M = 2-Methyl-4-hydroxy-4 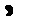 -nitrobenzophenon** N = 2-Methyl-4-hydroxy-3 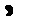 -nitrobenzophenon** O = 4-Hydroxy-4 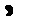 -nitrobenzophenon** P = 3,4-Dihydroxy-4 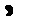 -nitrobenzophenon** Q = 3,4-Dihydroxy-3 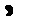 -nitrobenzophenon** | | | | | |

| | | | | | |
|---|---|---|---|---|---|
| * Bezogen von der Fa. Aldrich GmbH & Co KG, Steinheim. | | | | | |
| ** Nach einer gängigen Methode selbst hergestelltes Produkt (Friedel-Crafts-Acylierung) nach Houben-Weyl VII / 2a, 15) | | | | | |

### Beispiele 20 - 26:

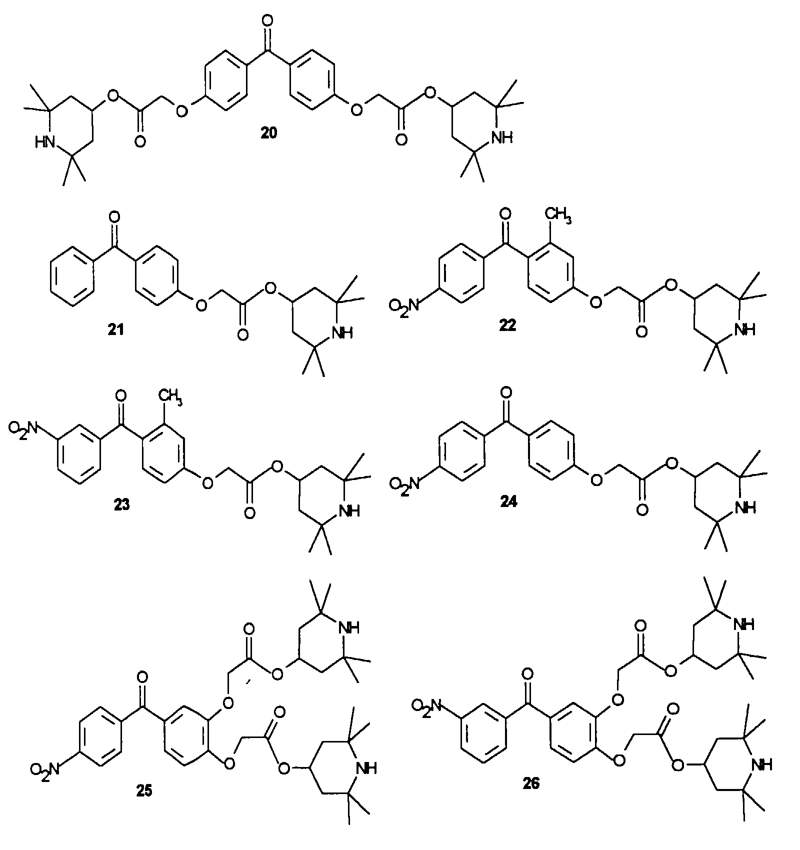

### Allgemeine Vorschrift: für die Herstellung der Verbindungen 20 - 26:

20 - 40 mmol des beschriebenen Methylesters (13 - 19), werden mit einer äquimolaren Menge 2,2,6,6-Tetramethylpiperidin-4-ol (vgl. Tabelle 4) und 200 mg Lithiumamid (9 mmol) in 200 ml Xylol bei 135°C über 8 h gerührt, wobei das langsam entstehende Methanol zusammen mit etwas Xylol langsam kontinuierlich abdestilliert wird. Die Mischung wird nach Erkalten in Wasser eingegossen. Die organische Phase wird dreimal mit je 50 ml Wasser gewaschen, mit Natriumsulfat getrocknet und von diesem abfiltriert. Nach Entfernen der flüchtigen Bestandteile im Vakuum wird das Produkt bei 80°C im Vakuum getrocknet.

**Tabelle 4**

| Versuchs-Nr. | 1. Edukt | 2. Edukt* | Ausbeute | Charakterisierung | λₘₐₓ** |
|---|---|---|---|---|---|
| **20** (M = 608,90) | **13**, 9,0 g (25 mmol) | 7,9 g (50 mmol) | 15,0 g (99%) | farblos, krist. Fp. = 125-128°C | 286 nm (vs) 330 nm (m, sh) |
| **21** (M = 395,54) | **14**, 18,8 g (40 mmol) | 6,3 g (40 mmol) | 14,9 g (95%) | hellgelb, krist. Fp. = 79-80°C | 284 nm (vs) 340 (m, sh) |
| **22** (M = 453,56)) | **15**, 13,1 g (40 mmol) | 6,3 g (40 mmol) | 13,6 g (75%) | hellgelb, krist. Fp. = 99-101°C | 266 nm (vs) 305 nm (s, sh) |
| **23** (M = 454,57) | **16**, 13,2 g (40 mmol) | 6,3 g (40 mmol) | 15,3 g (84%) | oranges Öl, It. ¹H NMR rein | 272,290 nm(vs) 350 nm (s) |
| **24** (M = 440,54)) | **17**, 12,6 g (40 mmol) | 6,3 g (40 mmol) | 16,0 g (91%) | gelb, kristallin Fp.= 125-126°C | 275 nm (vs) 295 nm (s, sh) |
| **25** (M = 653,85) | **18**, 10,1 g (25 mmol) | 7,9 g (50 mmol) | 13,8 g (84%) | beige, kristallin Fp.=128-131°C | 278,320 nm(vs) 450 nm (w, sh) |
| **26** (M = 653,85) | **19**, 14,5 g (36 mmol) | 11,3 g (72 mmol) | 5,3 g (22%) | braunes Öl, It. ¹H NMR rein | 275 nm (vs) 310 nm (s) |

| | | | | | |
|---|---|---|---|---|---|
| * 2,2,6,6-Tetramethylpiperidin-4-ol, bezogen von der Fa. HÜLS AG, Marl. | | | | | |
| ** Verbindungen 20, 21 wurden in Ethanol, Verbindungen 22, 24, 25 in DMSO, Verbindungen 23, 26 in CHCl₃ gemessen. | | | | | |

### Beispiel 27: Lichtstabilisierende Wirkung in Polypropylenfolien

100 Gewichtsteile unstabilisiertes Polypropylen ®Hostalen PPK wurden zusammen mit 0,1 Gewichtsteilen Calciumstearat, 0,05 Gewichtsteilen Pentaerythrityl-tetrakis-3-(3,5-di-t-butyl-4-hydroxyphenyl)-propionat und 0,1 Gewichtsteilen des zu prüfenden Stabilisators in einem Brabender-Mischer 10 min bei 200°C und 20 U/min geknetet. Aus dieser Mischung wurde bei 190°C eine 100 µm starke Folie gepreßt und die auf diese Weise erhaltenen Prüfkörper in einem Bewitterungsgerät (®Xenotest 1200) über die Dauer von 500 Stunden belichtet. Als Kriterium der Stabilität der Folie wurde die Veränderung des Carbonylindexes innerhalb dieser Zeitspanne herangezogen. Der Carbonylindex CO wurde hierbei gemäß der Formel CO = E₁₇₂₀/E₂₀₂₀ (E bedeutet die Extinktion bei der jeweiligen Wellenlänge) bestimmt. Zu Vergleichszwecken wurde eine Folie unter den gleichen Bedingungen, jedoch ohne den Zusatz eines erfindungsgemäßen Stabilisators getestet. Die Versuchsergebnisse sind in Tabelle 5 zusammengestellt:

**Tabelle 5**

| Zunahme des Carbonylindexes nach 500 Stunden Belichtung: | |
|---|---|
| Stabilisator | Δ(CO) |
| Bis(2,2,6,6-tetramethyl-4-piperidyl)sebacat | 0,02 |
| aus Beispiel 20 | 0,20 |
| aus Beispiel 25 | 0,23 |
| keiner | (400 h:2,0)* |

| | |
|---|---|
| * bereits nach 400 h beginnt die Folie so stark zu verspröden, daß der Versuch abgebrochen werden muß. | |

Tabelle 5 unterstreicht die sehr gute Lichtstabilität von erfindungsgemäß stabilisiertem Polypropylen gegenüber nicht stabilisiertem Polypropylen, wobei fast die Lichtstabilität von mit Bis(2,2,6,6-tetramethyl-4-piperidyl)sebacat stabilisiertem Polyproylen erreicht wird.

### Beispiel 28: Lichtstabilisierende Wirkung in Polypropylenfolien mit Extraktion

100 Gewichtsteile unstabilisiertes Polypropylen ®Hostalen PPK wurden zusammen mit 0,1 Gewichtsteilen Calciumstearat, 0,05 Gewichtsteilen Pentaerythrityl-tetrakis-3-(3,5-di-t-butyl-4-hydroxyphenyl)-propionat und 0,1 Gewichtsteilen des zu prüfenden Stabilisators in einem Brabender-Mischer 10 min bei 200°C und 20 U/min geknetet. Aus dieser Mischung wurde bei 190°C eine 100 µm starke Folie gepreßt und die auf diese Weise erhaltenen Prüfkörper in einem Bewitterungsgerät (®Xenotest 1200) 200 Stunden belichtet. Nach dieser Vorbelichtung wurden die Folienstücke mit Methylenchlorid extrahiert (24 h, 40°C) und anschließend über einen Zeitraum von weiteren 300 Stunden belichtet. Als Kriterium der Stabilität der Folie wurde die Veränderung des Carbonylindexes innerhalb dieser Zeitspanne (500 h Gesamtbeschichtungszeit) herangezogen. Der Carbonylindex CO wurde hierbei gemäß der Formel CO = E₁₇₂₀/E₂₀₂₀ bestimmt. Zu Vergleichszwecken wurde eine Folie unter den gleichen Bedingungen, jedoch ohne den Zusatz eines erfindungsgemäßen Stabilisators getestet. Die Versuchsergebnisse sind in Tabelle 6 zusammengestellt:

**Tabelle 6**

| Zunahme des Carbonylindexes nach 200 h Vorbelichtung, Extraktion mit Methylenchlorid (24 h, 40°C) und 300 Stunden Weiterbelichtung (ergibt 500 h Gesamtbelichtungszeit): | |
|---|---|
| Stabilisator | Δ(CO) |
| Bis(2,2,6,6-tetramethyl-4-piperidyl)sebacat | 1,08 |
| aus Beispiel 20 | 0,72 |
| aus Beispiel 25 | 0,86 |
| keiner | (400 h: 2,0)* |

| | |
|---|---|
| * bereits nach 400 h (200 h Vorbelichtung + 200 h Weiterbelichtung) beginnt die Folie so stark zu verspröden, daß der Versuch abgebrochen werden muß. | |

Tabelle 6 beweist, daß die Lichtschutzwirkung der erfindungsgemäß hergestellten Stabilisatoren auch nach Extraktion mit Methylenchlorid erhalten bleibt, und besser ist als Bis(2,2,6,6-tetramethyl(4-piperidyl)sebacat.

### Beispiel 29: Extraktionsverhalten der Stabilisatoren in Polypropylenfolien nach Belichtung:

Die analog Beispiel 27 hergestellten Prüfkörper wurden 200 h in einem Bewitterungsgerät (®Xenotest 1200) belichtet. Danach wurden die Prüfkörper einer Heißextraktion mit Methylenchlorid unterzogen und der in der Folie verbliebene, nicht extrahierbare Restgehalt an Stabilisator über eine Stickstoffbestimmung (nach Chemilumineszenzverfahren) bestimmt. Vor Beginn der Belichtung war unter den verwendeten Extraktionsbedingungen der überwiegende Anteil an Stabilisator mit Methylenchlorid aus den Prüfkörpern extrahierbar. Die Versuchsergebnisse sind in Tabelle 7 zusammengefaßt.

**Tabelle 7**

| Erschöpfende Heißextraktion der PP-Folien mit Methylenchlorid nach 200h Vorbelichtung | | | |
|---|---|---|---|
| Stabilisator | Menge in der Folie zu Beginn [%] | Menge nach 200h Belichtung und Extraktion * [%] | nicht extrahierbare Stabilisatormenge |
| Vergleich | 0,1 | 0,028 | 28% |
| Verbindung 1 | 0,1 | 0,067 | 67% |
| Verbindung 6 | 0,1 | 0,058 | 58% |
| Verbindung 20 | 0,1 | 0,071 | 71% |
| Verbindung 22 | 0,1 | 0,061 | 61% |
| Verbindung 25 | 0,1 | 0,067 | 67% |
| Vergleich = Bis(2,2,6,6-tetramethyl-4-piperidyl)sebacat | | | |

| | | | |
|---|---|---|---|
| * Extraktion mit Methylenchlorid, (24 h, 40°C) | | | |

Die erfindungsgemäßen Stabilisatoren sind nach 200 h Belichtung durch die dabei stattfindende Molgewichtsveränderung mit Methylenchlorid deutlich weniger gut extrahierbar als vor der Belichtung. Der Vergleichsversuch zeigt, daß die Stabilisatoren, die den Stand der Technik darstellen, fast vollständig extrahierbar sind.

## Patentansprüche

1. Neue UV-Stabilisatoren, dadurch gekennzeichnet, daß ihre Migrationsgeschwindigkeit in oder ihre Extrahierbarkeit aus organischen Materialien durch die Einwirkung von UV-Licht deutlich verringert wird.

2. Neue Stabilisatoren gemäß Anspruch 1 der allgemeinen Formel (I), 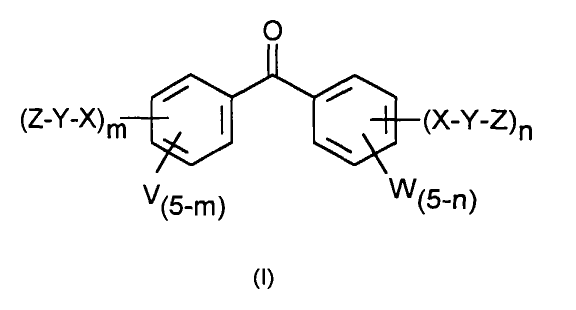 wobei
die im aromatischen Ring zur Carbonylgruppe α-ständigen Substituenten nicht OH bedeuten dürfen,
m und n unabhängig voneinander 0,1 oder 2 bedeuten,
V oder W unabhängig voneinander H, Hal, NO₂, OH, OR¹, CN, SR¹, C₁-C₁₈-Alkyl, C₃-C₁₀-Cycloalkyl, unsubstituiertes oder mit OH, OR¹, Hal oder NR¹R² substituiertes C₆-C₁₄-Aryl oder C₇-C₁₀-Arylalkyl, einen heteroaromatischen Rest mit 5-15 C-Atomen, NR¹R² oder COOR¹ bedeuten,
R¹ und R² H, C₁-C₁₈-Alkyl, C₃-C₁₀-Cycloalkyl, unsubstituiertes oder mit OH, OR¹ oder Hal substituiertes C₆-C₁₄-Aryl oder C₇-C₁₀-Arylalkyl, oder einen heteroaromatischen Rest mit 5-15 C-Atomen bedeuten,
X einen zweibindigen Rest -O-, -S- oder -C(O)- bedeutet,
Y für X = O oder S -CH₂-C(O)-, -C(O)-, -C(O)-C(O)-, -CH₂-CHR¹-, -(CH₂- CH₂O)ₙ- (mit n = 1 - 8) oder 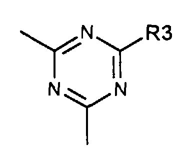 bedeutet,
wobei R³ NR¹R², OR¹, Halogen oder Z bedeutet,
Y für X = -C(O)- eine direkte Bindung zu Z bedeutet,
Z einen der folgenden Reste 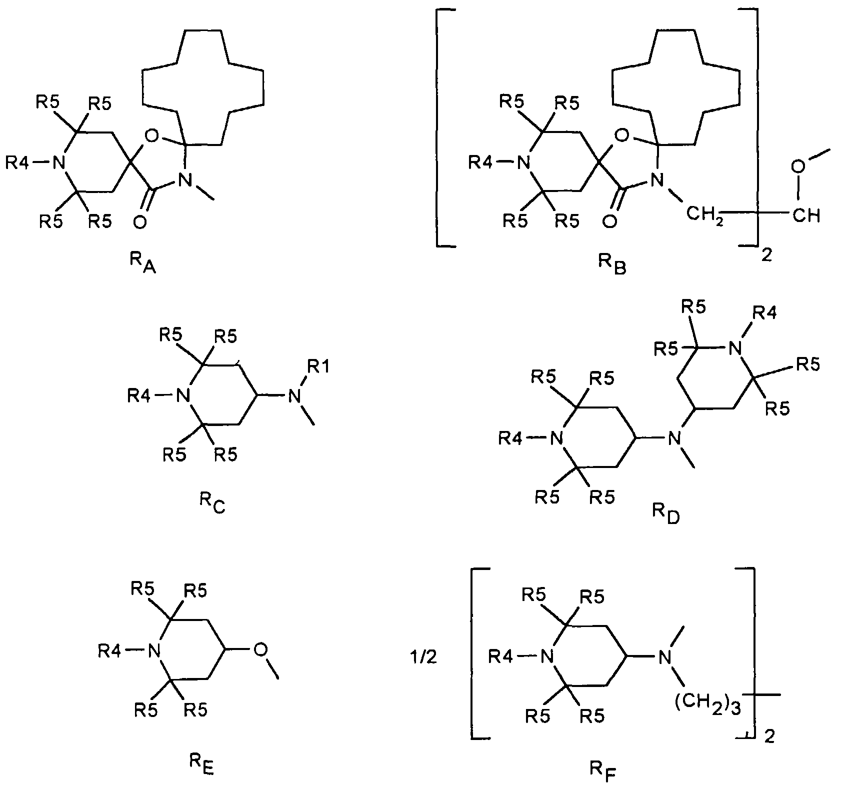 bedeutet,
worin
R⁴ Wasserstoff, C₁-C₂₀-Alkyl, ein Oxyl-Radikal, OH, NO, CH₂CN, unsubstituiertes oder mit OH, OR¹, Hal oder NR¹R² substituiertes C₆-C₁₄-Aryl oder C₇-C₁₀-Arylalkyl, Allyl, C₁-C₃₀-Alkyloxy, C₅-C₁₂-Cycloalkyloxy, C₃-C₁₀-Alkenyl, C₃-C₆-Alkinyl, C₁-C₁₀-Acyl, Halogen, und
R⁵ Wasserstoff oder C₁-C₄-Alkyl bedeutet.

3. Stabilisatoren gemäß Anspruch 1 oder 2, dadurch gekennzeichnet, daß
die im aromatischen Ring zur Carbonylgruppe α-ständigen Substituenten nicht OH bedeuten dürfen,
m und n unabhängig voneinander 0, 1 oder 2 bedeuten,
V oder W unabhängig voneinander H, NO₂, OH, C₁-C₁₀-Alkyl, C5-C₆-Cycloalkyl, unsubstituiertes oder mit OH oder NR¹R² substituiertes C₆-C₁₄-Aryl oder C₇-C₁₀-Arylalkyl, einen heteroaromatischen Rest mit 6-10 C-Atomen, NR¹R² oder COOR¹ bedeuten,
R¹ und R² H, C₁-C₁₀-Alkyl, C5-C₆-Cycloalkyl, unsubstituiertes oder mit OH substituiertes C₆-C₁₄-Aryl oder C₇-C₁₀-Arylalkyl, oder einen heteroaromatischen Rest mit 6-10 C-Atomen bedeuten,
X einen zweibindigen Rest -O- oder -C(O)- bedeutet,
Y für X = O -CH₂-C(O)-, -C(O)- oder -CH₂-CHR¹- bedeutet,
Z den Rest R_{A}, R_{C}, R_{D} oder R_{E} bedeutet,
R⁴ Wasserstoff, C₁-C₁₀-Alkyl, ein Oxyl-Radikal, OH, NO, unsubstituiertes oder mit OH oder NR¹R² substituiertes C₆-C₁₄-Aryl oder C₇-C₁₀-Arylalkyl, Allyl, C₁-C₁₀-Alkyloxy, C₆-C₉-Cycloalkyloxy, C₄-C₈-Alkenyl, C₃-C₆-Alkinyl, C₁-C₅-Acyl, Halogen, und
R⁵ Wasserstoff oder C₁-C₄-Alkyl bedeutet.

4. Stabilisatoren gemäß Anspruch 1 oder 2, dadurch gekennzeichnet, daß
die im aromatischen Ring zur Carbonylgruppe α-ständigen Substituenten nicht OH bedeuten dürfen,
m und n unabhängig voneinander 0, 1 oder 2 bedeuten,
V oder W unabhängig voneinander H, NO₂, C₁-C₄-Alkyl, NR¹R², oder COOR¹ bedeuten,
X einen zweibindigen Rest -O- oder -C(O)- bedeutet,
Y für X = -O- -C(O)- oder CH₂-C(O)- bedeutet,
Y für X = -C(O)- eine direkte Bindung zu Z bedeutet und
Z einen Rest der Formel R_{C} oder R_{E} bedeutet, worin
R⁴ Wasserstoff, OH, NO, C₁-C₅-Alkyl, C₁-C₅-Alkyloxy, C₁-C₅-Acyl und
R⁵ Methyl bedeutet.

5. Verfahren zur Herstellung der Verbindungen der Formel (I) gemäß Anspruch 2, dadurch gekennzeichnet, daß ein Benzophenon (II) mit einem oder mehreren Äquivalenten eines geeigneten Reagenzes R in ein reaktives Molekül (III) überführt wird, 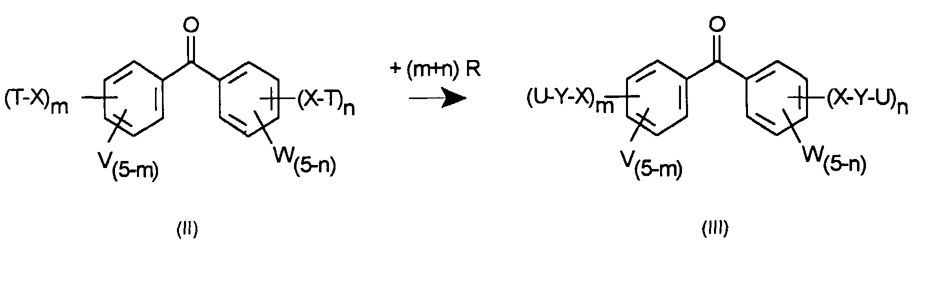 wobei
V, W, X und Y die in Formel (I) angegebene Bedeutung aufweisen,
T für X = -O- oder -S- Wasserstoff bedeutet,
T für X = -C(O)- OR¹, NR¹R², -O-(CO)-R¹, OH bedeutet,
R U-Y-U, Thionylchlorid, Phosphor(III)halogenid, Halogenwasserstoff, elementares Chlor bedeutet, wobei U Wasserstoff, Halogen, OR¹ oder NR¹R² bedeutet, und
wobei in einem zweiten Schritt die Verbindung (III) mit Z-H zu der erfindungsgemäßen Verbindung (I) umgesetzt wird, wobei H für Wasserstoff steht und Z die in Anspruch 2 definierten Bedeutung besitzt.

6. Verfahren gemäß Anspruch 5, dadurch gekennzeichnet, daß es sich bei dem Reagenz R um 2,4,6-Trichlor-s-triazin, Phosgen, Bromessigsäurealkylester, Chloressigsäurealkylester, Chlorameisensäuremethylester, Oxalsäure, Oxalsäuredihalogenid, Oxalsäuredialkylester, Oxalsäurehalogenidmonoester, Ethylenoxid, Thionylchlorid, Phosphor(III)halogenid, Halogenwasserstoff oder elementares Chlor handelt.

7. Verfahren gemäß Anspruch 5, dadurch gekennzeichnet, daß die Umsetzung in einem protischen oder aprotischen, organischen Lösemittel, vorzugsweise einem Kohlenwasserstoff, insbesondere aromatischen Kohlenwasserstoffen wie Toluol, Xylol oder in Gemischen hieraus oder in Tetrahydrofuran erfolgt.

8. Verfahren gemäß Anspruch 5, dadurch gekennzeichnet, daß eine der Reaktionskomponenten im Überschuß als Lösemittel verwendet wird.

9. Verfahren zur Herstellung der Verbindungen der Formel (I) gemäß Anspruch 2, dadurch gekennzeichnet, daß zuerst eine der Verbindungen Z-H oder Z-M mit M = Alkali, mit einem Reagenz U-Y-U zu dem Intermediat Z-Y-U umgesetzt wird und daß die Verbindung Z-Y-U in einer anschließenden Reaktion mit einem geeignet substituierten Derivat des Benzophenons der Formel (II) mit -XT = -OH oder -SH zu den Verbindungen der Formel (I) umgesetzt wird.

10. Verfahren gemäß Anspruch 9, dadurch gekennzeichnet, daß zur Reaktion das Anion Z⁻ verwendet wird, welches vorzugsweise aus der Verbindung Z-H hergestellt wird, wobei Z die in Formel (I) beschriebene Bedeutung besitzt und H Wasserstoff bedeutet.

11. Verfahren zum Stabilisieren von polymeren Material gegen den Abbau durch Licht, Strahlung, Wärme und Sauerstoff, dadurch gekennzeichnet, daß man dem zu stabilisierenden Material eine Verbindung der Formel (I) nach Anspruch 2 in einer Konzentration von 0,001-5 Gew.%, bevorzugt von 0,1-2,0 Gew.% zusetzt.

12. Verfahren gemäß Anspruch 11, dadurch gekennzeichnet, daß Antioxidantien, Lichtstabilisatoren, Metalldesaktivatoren, Antistatika, flammhemmende Mittel, Pigmente oder Füllstoffe als weitere Additive eingesetzt werden.
